# DEMANDE DE BREVET EUROPEEN

(11) **EP 4 756 139 A1**
(43) Date de publication de la demande: **10.06.2026**
(21) Numéro de dépôt: 24218395.2
(22) Date de dépôt: 09.12.2024
(51) Int. Cl.: E03B 7/07, G01N 33/00, G06Q 50/06

(54) **SYSTÈME DE GESTION INTELLIGENTE DE L'EAU AVEC VANNE MOTORISÉE, CAPTEURS AVANCÉS ET CONNECTIVITÉ ÉTENDUE**

(71) Demandeur: Adda-Benikhlef, Mehdi, 1271 Givrins (CH)
(72) Inventeur: Adda-Benikhlef, Mehdi, 1271 Givrins (CH)
(74) Mandataire: Weihs, Bruno Konrad

(57) **Abrégé**

La présente invention concerne un système (2) de gestion de l'eau pour un réseau de distribution de l'eau comprenant au moins un point d'entrée de l'eau (22) et au moins un point de distribution de l'eau (24), comprenant : des moyens de détection (4) placé entre l'au moins un point d'entrée de l'eau (22) et l'au moins un point de distribution de l'eau (24) et configurés pour mesurer un débit de l'eau ; une vanne (26) configuré pour contrôler le débit de l'eau entre l'au moins un point d'entrée de l'eau (22) et l'au moins un point de distribution de l'eau (24) ; un module de contrôle (10) configuré pour : recevoir et analyser des données (DA) mesurées par les moyens de détection (4) ; détecter et signaler une ou plusieurs anomalies liées au débit de l'eau, et, donner un ordre (OR) à l'au moins une vanne (26) de modifier le débit d'eau en cas de détection d'anomalie.

## Description

### DOMAINE TECHNIQUE

La présente invention concerne la gestion de la consommation et de la distribution de l'eau dans un réseau de distribution de l'eau comprenant un ou plusieurs points de distribution de l'eau.

L'invention concerne plus particulièrement un système de gestion de l'eau comprenant des moyens de détection, une ou plusieurs vannes, et un module de contrôle, selon l'invention.

### ÉTAT DE LA TECHNIQUE

Dans un réseau de distribution de l'eau, un système de gestion de l'eau sert à contrôler la fourniture, la distribution, et l'utilisation de l'eau dans un endroit spécifique à petite et grande échelle, par exemple dans une ville, une maison, ou un immeuble d'appartement.

De tels systèmes peuvent comprendre par exemple des compteurs pour assurer la disponibilité et l'utilisation efficace de l'eau. La présence de ces compteurs permet par exemple de mesurer la consommation de l'eau à un point d'entrée de l'eau dans un réseau de distribution.

Malgré la présence de compteurs, de nombreux problèmes existent dans les systèmes de gestion de l'eau existants.

La plupart de ces systèmes contrôlent uniquement la consommation de l'eau, et cela, pour tout le réseau de distribution dans sa globalité, et non pas de manière plus localiser, par exemple, la consommation de chacun des points de sortie de l'eau. De plus, ces systèmes ne contrôlent pas en général des caractéristiques biologiques ou chimiques de l'eau.

La plupart de ces systèmes sont passifs, c'est-à-dire, ils peuvent alerter mais ne peuvent pas anticiper des pannes ni réagir en cas d'anomalie.

Ces systèmes sont souvent adaptés soit pour un environnement domestique soit pour un environnement industriel, et donc ne sont pas flexibles dans leurs champs d'application et contribuent à de l'inefficacité dans leur production et dans leur utilisation, nécessitant une familiarisation avec deux systèmes au lieu d'un seul système.

Ces systèmes sont le plus souvent destinés à des fins dites « monitoring », c'est-à-dire, de suivre la consommation de l'eau mais non à analyser sa consommation ainsi que d'autres paramètres d'un réseau de distribution de l'eau, donc ne permettent pas une compréhension plus profonde des caractéristiques du réseau ainsi que le flux ou les flux d'eau présents dans le réseau, en temps réel et par le passé.

Finalement, les systèmes de gestion de l'eau existants requièrent une alimentation de puissance soit externe, par branchement filaire, soit interne avec une batterie. Cela n'exploite pas l'énergie hydroélectrique présente grâce au flux de l'eau et laisse le système vulnérable à des coupures de courant ou d'oubli de changement de batterie épuisée.

La publication brevet EP3652151B1 divulgue un système pour la gestion de la consommation de l'eau selon l'état de la technique.

Il existe donc un besoin d'un système de gestion de l'eau intelligent et automatisé, c'est-à-dire capable de surveiller, analyser, en contrôler la consommation de l'eau, qui est universel et peut s'adapter à divers environnement domestiques et industriels.

Il existe également un besoin d'un système de gestion de l'eau qui peut réagir en cas d'anomalie, par exemple qui peut couper le flux de l'eau en cas de fuite ou qui peut alerter en cas de détection de contamination biologiques ou chimiques.

Il existe également un besoin d'un système de gestion de l'eau qui est autonome sur le plan énergétique, qui peut s'adapter son alimentation de puissance en fonction des sources d'énergie disponible.

Il existe donc un besoin d'un système de gestion du flux de l'eau amélioré pour répondre aux inconvénients de l'art antérieur.

### SOMMAIRE DE L'INVENTION

La présente invention permet de remédier aux inconvénients de l'art antérieur et concerne un système de gestion de l'eau pour un réseau de distribution de l'eau comprenant au moins un point d'entrée de l'eau et au moins un point de distribution de l'eau selon la revendication 1.

Il est proposé, selon un premier aspect de l'invention, un système de gestion de de l'eau pour un réseau de distribution de l'eau comprenant au moins un point d'entrée de l'eau et au moins un point de distribution de l'eau, comprenant : des moyens de détection placé entre l'au moins un point d'entrée de l'eau et l'au moins un point de distribution de l'eau et configurés pour mesurer un débit de l'eau ; au moins une vanne configurée pour contrôler le débit de l'eau entre l'au moins un point d'entrée de l'eau et l'au moins un point de distribution de l'eau ; un module de contrôle configuré pour : recevoir et analyser des données (DA) mesurées par les moyens de détection ; détecter et signaler une ou plusieurs anomalies liées au débit de l'eau, et, donner un ordre (OR) à l'au moins une vanne de modifier le débit d'eau en cas de détection d'anomalie.

Selon une variante de l'invention, l'une ou plusieurs anomalies peuvent comprendre au moins l'une de la liste d'anomalies comprenant une fuite, et une présence de débit de l'eau inattendu, par exemple causé par un voleur présent dans une zone comprenant le réseau de distribution de l'eau.

Selon une autre variante de l'invention, les moyens de détection peuvent en outre être configurés pour mesurer des propriétés chimiques et biologiques de l'eau, et le module de contrôle est en outre configuré pour détecter et signaler des anomalies liées aux propriétés chimiques et biologiques de l'eau.

Selon une autre variante de l'invention, le système de gestion de l'eau peut comprendre au moins un premier capteur placé entre l'au moins un point d'entrée de l'eau et les moyens de détection, et configuré pour mesurer le flux d'eau instantané entre le point d'entrée de l'eau et les moyens de détection et pour communiquer avec le module de contrôle.

Selon une autre variante de l'invention, le système de gestion de l'eau peut comprendre au moins un deuxième capteur placé entre les moyens de détection et l'au moins un point de sortie de l'eau, et configuré pour mesurer le flux d'eau instantané entre les moyens de détection et l'au moins un point de distribution de l'eau, et pour communiquer avec le module de contrôle.

Selon une autre variante de l'invention, le système de gestion de l'eau peut comprendre un module d'affichage mobile configuré pour communiquer avec le module de contrôle, générer une visualisation des caractéristiques associées au débit ou aux propriétés de l'eau, et notifier un utilisateur en cas d'anomalie détectée par le module de contrôle.

Selon une autre variante de l'invention, les moyens de détection peuvent comprendre au moins l'un de la liste de moyens de détection comprenant un débitmètre, un ou plusieurs capteurs intégrés pour mesurer une pression de l'eau, un pH de l'eau, et une concentration des contaminants biologiques et chimiques dans l'eau.

Selon une autre variante de l'invention, un actionnement de l'au moins une vanne peut comprendre un actionnement manuel ou motorisé.

Selon une autre variante de l'invention, le système de gestion de l'eau peut comprendre des moyens de récupération et de conversion de l'énergie hydroélectrique du débit de l'eau pour alimenter les besoins énergétiques du système. Les moyens de récupération et de conversion peuvent comprendre un générateur hydraulique pour recharger une batterie interne du système selon une variante de l'invention.

Selon une autre variante de l'invention, le module de contrôle peut être configuré choisir l'alimentation électrique du système entre la batterie interne rechargeable et un raccordement au réseau d'électricité.

Selon une autre variante de l'invention, les moyens de détection et le module de contrôle peuvent comprendre des moyens de communication sans fil, tels que le WiFi ou le Bluetooth, par exemple pour se communiquer.

Selon un deuxième aspect de l'invention, il est proposé une méthode de détection d'une ou plusieurs anomalies liées à un débit de l'eau dans un réseau de distribution de l'eau comprenant au moins un point d'entrée de l'eau et au moins un point de distribution de l'eau, comprenant : une mesure, entre l'au moins un point d'entrée de l'eau et l'au moins un point de distribution de l'eau, d'un débit de l'eau par des moyens de détection ; un contrôle du débit de l'eau entre l'au moins un point d'entrée de l'eau et l'au moins un point de distribution de l'eau par une vanne ; une réception et analyse des données (DA) mesurées par les moyens de détection, et une détection et un signalement d'une ou plusieurs anomalies liées au débit de l'eau par un module de contrôle, et une génération d'un ordre (OR) à l'au moins une vanne de modifier le débit d'eau en cas de détection d'anomalie.

### BRÈVE DESCRIPTION DES DESSINS

D'autre caractéristiques et avantages de la présente invention apparaitront plus clairement à la lecture de la description détaillée d'exemples de réalisation de l'invention et en références aux dessins annexés dans lesquels :
la Figure 1 représente l'ensemble d'un système de gestion d'eau selon une variante de l'invention ;
la Figure 2 représente l'ensemble du système de gestion de l'eau sous forme de schématique selon une autre variante de l'invention ;
la Figure 3 représente un module de contrôle et un module d'affichage intégrés selon une variante de l'invention ; et
la Figure 4 représente une méthode de détection d'une ou plusieurs anomalies liées à un débit d'eau dans un réseau de distribution de l'eau selon une variante de l'invention.

Les mêmes références seront utilisées pour référencer les mêmes éléments ou des éléments similaires dans l'ensembles des Figures.

### DESCRIPTION DÉTAILLÉE D'EXEMPLES DE RÉALISATION DE L'INVENTION

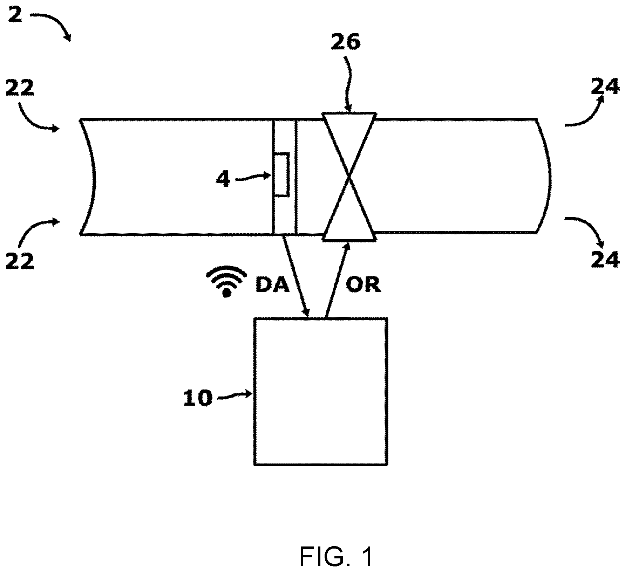

Le système de gestion de l'eau selon une variante de l'invention est représenté, par exemple, dans la Fig. 1.

Dans un premier aspect, l'invention comprend un système de gestion de l'eau 2 pour un réseau de distribution de l'eau comprenant au moins un point d'entrée de l'eau 22 et au moins un point de distribution de l'eau 24.

Le système de gestion de l'eau 2 peut être intégré dans un réseau de distribution de l'eau dans un environnement domestique ou industriel, par exemple, dans un réseau sous-terrain de ville ou dans un réseau de plomberie dans une maison, un immeuble d'appartement, ou un appartement individuel.

Comme illustré dans la Fig. 1, le système de gestion de l'eau 2 comprend des moyens de détection 4 placés entre l'au moins un point d'entrée de l'eau 22 et l'au moins un point de distribution de l'eau 24 et configurés pour mesurer un débit de l'eau, par exemple un débit d'un cours d'eau s'écoulant entre l'au moins un point d'entrée de l'eau 22 et l'au moins un point de distribution de l'eau 24, par exemple s'écoulant dans un tuyau du réseau de distribution.

L'au moins un point d'entrée 22 de l'eau peut comprendre, par exemple, la jonction entre une ligne d'approvisionnement de l'eau entrante principale d'une maison ou d'un immeuble et la plomberie de la maison ou de l'immeuble.

L'au moins un point de distribution de l'eau 24 peut comprendre, par exemple, une pluralité de robinets dans une maison ou appartement.

Les moyens de détection 4 peuvent être placés plus près du au moins un point d'entrée de l'eau 22 que du au moins un point de distribution de l'eau 24, plus près du au moins un point de distribution de l'eau 24 que du au moins un point d'entrée de l'eau 24, ou équidistant entre les deux points 22 et 24.

Les moyens de détection 4 peuvent comprendre, par exemple, un ou une pluralité de capteurs, par exemple, au moins l'un d'une liste de capteurs comprenant un débitmètre, un capteur de débit ou de flux de l'eau, un pH-mètre, un capteur de pression de l'eau, et un capteur biologique configuré pour mesurer la concentration de contaminants dans l'eau. D'autres capteurs sont possibles, par exemple, des capteurs de température, turbidité, dureté, et radioactivité. Le choix de capteurs est configurable selon les besoins spécifiques du réseau de distribution de l'eau, les exemples sont donnés à des fins purement illustratives.

Le système de gestion de l'eau 2 comprend en outre une vanne 26 configurée pour contrôler le débit de l'eau entre l'au moins un point d'entrée de l'eau 22 et l'au moins un point de distribution de l'eau 24.

L'au moins une vanne 26 peut être de type à boisseau ou à papillon par exemple, et sa taille peut être adaptée en fonction du diamètre d'un tuyau dans lequel le système est destiné à être installé. L'au moins une vanne 26 peut être de construction selon les normes usuelles du domaine, par exemple ANSI ou ISO, et sa pression nominale (PN) de conception peut être choisie en fonction des besoins spécifiques du réseau. Des exemples non-limitatifs sont par exemple PN16, PN25, ou PN40. Plusieurs matières de construction peuvent être également choisies, par exemple, le laiton, le bronze, ou l'acier inoxydable. L'actionnement de l'au moins une vanne 26 peut être manuel ou motorisé, par exemple en utilisant une électrovanne, ou des moyens hydrauliques ou pneumatiques.

Comme illustré dans la Fig. 1, le système de gestion de l'eau 2 comprend en outre un module de contrôle 10 configuré pour recevoir et analyser des données (DA) mesurées par les moyens de détection 4, et pour détecter et signaler une ou plusieurs anomalies liées au débit de l'eau, et pour donner un ordre (OR) à l'au moins une vanne 26 de modifier le débit d'eau en cas de détection d'anomalie.

L'au moins une vanne 26 peut être configurée pour communiquer avec le module de contrôle 10, par exemple, l'au moins une vanne 26 peut être commandée à partir d'un signal de commande transmis par le module de contrôle 10. L'au moins une vanne 26 peut comprendre par exemple une antenne configurée pour recevoir un tel signal de commande et des moyens pour convertir le signal en un actionnement manuel ou motorisé de l'au moins une vanne 26, par exemple à l'aide d'un servomoteur électrique en cas d'actionnement motorisé.

Le module de contrôle 10 peut comprendre, par exemple, un microcontrôleur comprenant un processeur, de la mémoire, des unités périphériques et des interfaces d'entrée-sortie, et configuré pour le traitement des données mesurées par les moyens de détection 4, à l'aide d'un programme d'ordinateur qui analyse les données pour détecter des anomalies.

Le module de contrôle 10 peut commander le comportement ou l'actionnement de la vanne 26 à distance, le module de contrôle 10 n'a pas besoin d'être à proximité de l'au moins une vanne 26. Le module de contrôle 10 peut être localisé dans un centre d'opération du réseau de l'eau, par exemple.

Selon une variante de l'invention, le module de contrôle 10 peut, par exemple, à partir des données mesurées par les moyens de détection 4, détecter une fuite dans un tuyau dans lequel le système 2 opère, sonner une alarme pour signaler la fuite, et commander l'au moins une vanne 26 d'arrêter le flux de l'eau dans le tuyau.

Ce mode de réalisation a pour avantage de prévenir des dommages causés par les fuites d'eau dans des immeubles, sans nécessiter l'intervention d'un opérateur.

Selon une autre variante de l'invention, le module de contrôle 10 peut, par exemple, être configuré dans un mode anti-vol, dans lequel le module de contrôle 10 sonne une alarme quand un débit de l'eau inattendu est détecté, par exemple causé par un voleur qui ouvre un robinet ou tire la chasse dans une zone comprenant le réseau de distribution de l'eau dans lequel le système de gestion de l'eau 2 est installé. Dans ce mode, un utilisateur du système peut choisir une configuration dans laquelle une alarme sonne si un débit d'eau au-dessus d'une limite volumétrique configurable est détecté dans un tuyau dans lequel il est connu que l'eau ne devrait pas couler pendant une durée d'absence de l'utilisateur de la zone comprenant le réseau.

Ce mode de réalisation a pour avantage d'alerter un utilisateur du système de gestion de l'eau 2 en cas de présence présumée d'un voleur dans une zone comprenant le réseau de distribution de l'eau, par exemple un propriétaire d'une maison où le système de gestion de l'eau 2 est installé pendant les vacances du propriétaire.

Selon une autre variante de l'invention, les moyens de détection 4 peuvent comprendre des capteurs configurés pour mesurer des propriétés chimiques et biologiques de l'eau, et le module de contrôle 10 peut être configuré pour signaler des anomalies biologiques ou chimiques de l'eau, par exemple à l'aide d'un signal sonore distinct de l'alarme de fuite ou de vol.

Ce mode de réalisation a pour avantage d'alerter un utilisateur du système en cas de contamination biologique ou chimique de l'eau, donc de permettre à l'utilisateur de chercher la source de la contamination et de mettre en place un processus de décontamination.

Le système de gestion de l'eau 2 peut, en option, comprendre un module d'affichage mobile 12 configuré pour communiquer avec le module de contrôle 10, générer une visualisation des caractéristiques associées au débit ou aux propriétés de l'eau, et notifier un utilisateur en cas d'anomalie.

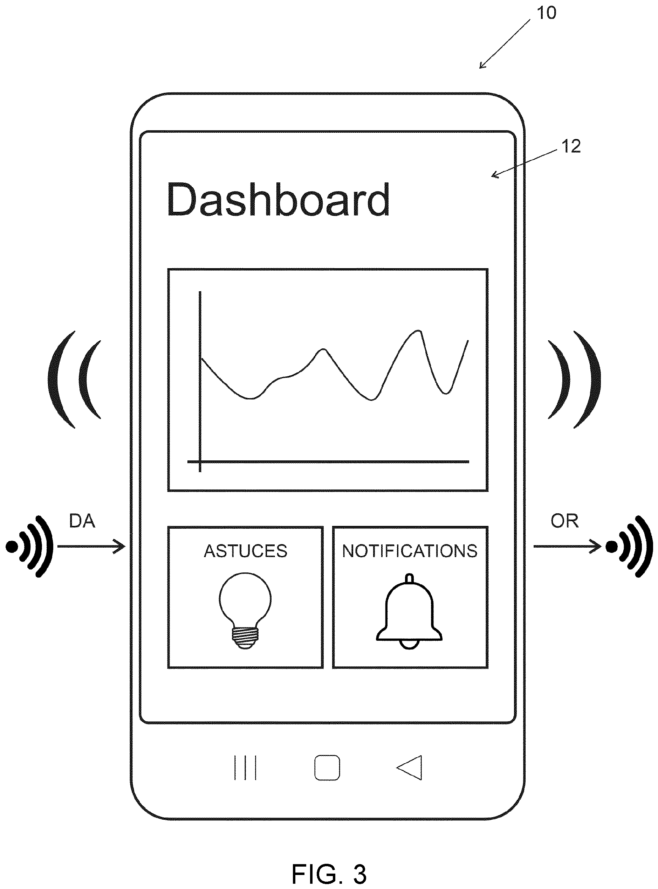

Selon une autre variante de l'invention illustré dans la Fig. 3, le module d'affichage mobile 12 peut être une application mobile, par exemple, installée sur un smartphone ou autre appareil mobile. Il peut être configuré pour, par exemple, afficher la consommation de l'eau en temps réel, donner des suggestions à l'utilisateur pour réduire sa consommation de l'eau, et signaler une fuite, un vol, ou une contamination biologique ou chimique. Selon une autre variante de l'invention, le module de contrôle 10 peut être intégré dans un smartphone ou autre appareil mobile comprenant le module d'affichage mobile 12.

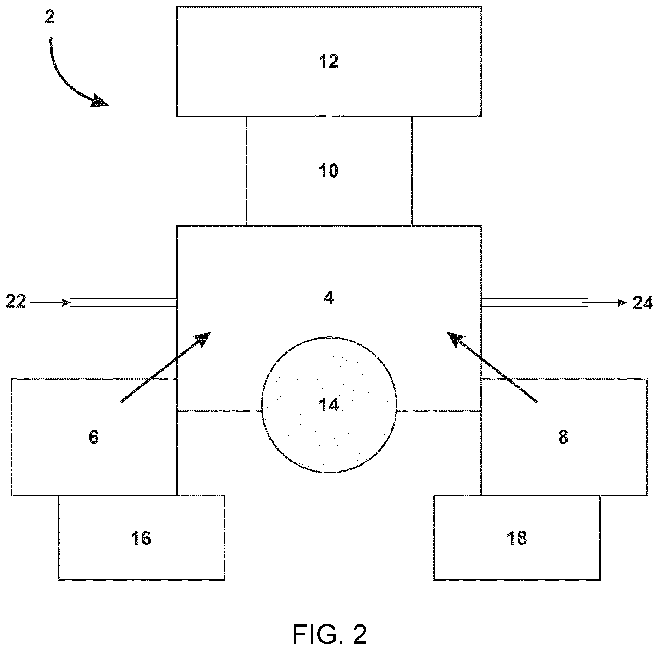

Selon une autre variante de l'invention illustré dans la Fig. 2, le système de gestion de l'eau 2 peut comprendre des moyens de récupération et de conversion 14 de l'énergie hydroélectrique du débit de l'eau pour alimenter les besoins énergétiques du système 2.

Les moyens de récupération et de conversion peuvent comprendre, par exemple, un générateur hydraulique configuré pour recharger une batterie interne du système 16.

Ce mode de réalisation a pour avantage d'exploiter l'énergie hydroélectrique présente grâce au débit de l'eau, donc réduit la dépendance du système sur des ressources externes d'énergie et garantit le fonctionnement du système même en cas de panne d'électricité. Cet mode de réalisation a également pour avantage de réduire la fréquence à laquelle des batteries épuisées doivent être remplacées.

En outre, selon un mode de réalisation également illustré dans la Fig. 2, le module de contrôle peut être configuré pour choisir l'alimentation électrique du système entre la batterie interne rechargeable 16 ou un raccordement au réseau d'électricité 18, permettant un mode d'opération hybride selon les conditions actuelles de réseau d'électricité et du système.

Le système peut en option comprendre en outre un ou plusieurs capteurs à l'extérieur des moyens de détection 4, par exemple un premier capteur 6 placé entre l'au moins un point d'entrée de l'eau 22 et les moyens de détection 4, et configuré pour mesurer le flux d'eau instantané entre le point d'entrée de l'eau et les moyens de détection 4 et pour communiquer avec le module de contrôle 10, et un deuxième capteur 8 placé entre les moyens de détection 4 et l'au moins un point de sortie de l'eau 24, et configuré pour mesurer le flux d'eau instantané entre les moyens de détection 4 et l'au moins un point de distribution de l'eau et pour communiquer avec le module de contrôle 10. Un ou plusieurs capteurs sont possible.

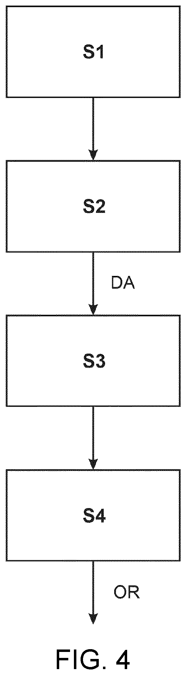

Dans un deuxième aspect illustré dans la Fig. 4, l'invention comprend une méthode de détection d'une ou plusieurs anomalies liées à un débit de l'eau dans un réseau de distribution de l'eau comprenant au moins un point d'entrée de l'eau 22 et au moins un point de distribution de l'eau 24, comprenant une mesure (S1), entre l'au moins un point d'entrée de l'eau 22 et l'au moins un point de distribution de l'eau 24, d'un débit de l'eau par des moyens de détection 4 ; un contrôle (S2) du débit de l'eau entre l'au moins un point d'entrée de l'eau 22 et l'au moins un point de distribution de l'eau 24 par une vanne 26 ; une réception et analyse des données (S3) (DA) mesurées par les moyens de détection 4, et la détection et signalement d'une ou plusieurs anomalies liées au débit de l'eau par un module de contrôle 10, et une génération (S4) d'un ordre (OR) ou d'une commande à l'au moins une vanne 26 de modifier le débit d'eau en cas de détection d'anomalie, par un module de contrôle 10.

On comprendra que diverses modifications et/ou améliorations évidentes pour l'homme du métier peuvent être apportées aux différents modes de réalisation de l'invention décrits dans la présente description sans sortir du cadre de l'invention défini par les revendications annexées.

L'invention ne se limite évidemment pas aux exemples illustrés dans le présent texte de description.

## Revendications

1. Un système de gestion de d'eau (2) pour un réseau de distribution de l'eau comprenant au moins un point d'entrée de l'eau (22) et au moins un point de distribution de l'eau (24), comprenant :
- des moyens de détection (4) placé entre l'au moins un point d'entrée de l'eau (22) et l'au moins un point de distribution de l'eau (24) et configurés pour mesurer un débit de l'eau ;
- au moins une vanne (26) configurée pour contrôler le débit de l'eau entre l'au moins un point d'entrée de l'eau (22) et l'au moins un point de distribution de l'eau (24) ;
- un module de contrôle (10) configuré pour :
∘ recevoir et analyser des données (DA) mesurées par les moyens de détection (4) ;
∘ détecter et signaler une ou plusieurs anomalies liées au débit de l'eau, et,
∘ donner un ordre (OR) à l'au moins une vanne (26) de modifier le débit d'eau en cas de détection d'anomalie.

2. Le système de gestion de l'eau (2) selon la revendication précédente, dans lequel l'une ou plusieurs anomalies comprennent au moins l'une de la liste d'anomalies comprenant une fuite, et une présence de débit de l'eau inattendu, par exemple causé par un voleur présent dans une zone comprenant le réseau de distribution de l'eau.

3. Le système de gestion de l'eau (2) selon l'une quelconque des revendications précédentes, **caractérisé en ce que** les moyens de détection (4) sont en outre configurés pour mesurer des propriétés chimiques et biologiques de l'eau, et le module de contrôle (10) est en outre configuré pour détecter et signaler des anomalies liées aux propriétés chimiques et biologiques de l'eau.

4. Le système de gestion de l'eau (2) selon l'une quelconque des revendications précédentes, **caractérisé en ce qu'**il comprend au moins un premier capteur (6) placé entre l'au moins un point d'entrée de l'eau (22) et les moyens de détection (4), et configuré pour mesurer le flux d'eau instantané entre le point d'entrée de l'eau et les moyens de détection (4) et pour communiquer avec le module de contrôle (10).

5. Le système de gestion de l'eau (2) selon l'une quelconque des revendications précédentes, **caractérisé en ce qu'**il comprend au moins un deuxième capteur (8) placé entre les moyens de détection (4) et l'au moins un point de sortie de l'eau (24), et configuré pour mesurer le flux d'eau instantané entre les moyens de détection (4) et l'au moins un point de distribution de l'eau, et pour communiquer avec le module de contrôle (10).

6. Le système de gestion de l'eau (2) selon l'une quelconque des revendications précédentes, **caractérisé en ce qu'**il comprend un module d'affichage mobile (12) configuré pour communiquer avec le module de contrôle (10), générer une visualisation des caractéristiques associées au débit ou aux propriétés de l'eau, et notifier un utilisateur en cas d'anomalie détectée par le module de contrôle.

7. Le système de gestion de l'eau (2) selon l'une quelconque des revendication précédentes 3 à 6, **caractérisé en ce que** les moyens de détection (4) comprennent au moins l'un de la liste de moyens de détection comprenant un débitmètre, un ou plusieurs capteurs intégrés pour mesurer une pression de l'eau, un pH de l'eau, et une concentration des contaminants biologiques et chimiques dans l'eau.

8. Le système de gestion de l'eau (2) selon l'une quelconque des revendications précédentes, **caractérisé en ce qu'**un actionnement de l'au moins une vanne (26) comprend un actionnement manuel ou motorisé.

9. Le système de gestion de l'eau (2) selon l'une quelconque des revendications précédentes, **caractérisé en ce qu'**il comprend des moyens de récupération et de conversion (14) de l'énergie hydroélectrique du débit de l'eau pour alimenter les besoins énergétiques du système (2).

10. Le système de gestion de l'eau (2) selon la revendication précédente, **caractérisé en ce que** les moyens de récupération et de conversion (14) de l'énergie hydroélectrique comprennent un générateur hydraulique configuré pour recharger une batterie interne du système (16).

11. Le système de gestion de l'eau (2) selon l'une quelconque des revendications 10 ou 11, **caractérisé en ce que** le module de contrôle (10) est en outre configuré pour choisir l'alimentation électrique du système entre la batterie interne rechargeable et un raccordement au réseau d'électricité (18).

12. Le système de gestion de l'eau (2) selon l'une quelconque des revendications précédentes, dans lequel les moyens de détection (4), et le module de contrôle (10) comprennent des moyens de communications sans fil, tels que le WiFi ou le Bluetooth.

13. Une méthode de détection d'une ou plusieurs anomalies liées à un débit de l'eau dans un réseau de distribution de l'eau comprenant au moins un point d'entrée de l'eau (22) et au moins un point de distribution de l'eau (24), comprenant :
- une mesure, entre l'au moins un point d'entrée de l'eau (22) et l'au moins un point de distribution de l'eau (24), d'un débit de l'eau par des moyens de détection (4) ;
- un contrôle du débit de l'eau entre l'au moins un point d'entrée de l'eau (22) et l'au moins un point de distribution de l'eau (24) par une vanne (26) ;
- une réception et une analyse des données (DA) mesurées par les moyens de détection (4), et une détection et un signalement d'une ou plusieurs anomalies liées au débit de l'eau par un module de contrôle (10), et
- une génération d'un ordre (OR) à l'au moins une vanne (26) de modifier le débit d'eau en cas de détection d'anomalie.
